# FASCICULE DE BREVET EUROPEEN

(11) **EP 2 131 823 B1**
(45) Date de publication et mention de la délivrance du brevet: **30.03.2011**
(21) Numéro de dépôt: 08716926.4
(22) Date de dépôt: 19.02.2008
(51) Int. Cl.: A61K 9/70, A61K 31/454, A61P 1/08

(54) **FILM MONOCOUCHE A DESINTEGRATION RAPIDE POUR L'ADMINISTRATION BUCCALE DE SUBSTANCES ACTIVES**
SCHNELL ZERFALLENDE EINSCHICHTIGE FOLIE ZUR ORALEN VERABREICHUNG VON WIRKSTOFFEN
RAPID-DISINTEGRATION MONOLAYER FILM FOR THE ORAL ADMINISTRATION OF ACTIVE SUBSTANCES

(30) Priorité: 28.02.2007 FR 0753562
(43) Date de publication de la demande: 16.12.2009
(73) Titulaire: Pierre Fabre Medicament, 92100 Boulogne-Billancourt (FR)
(72) Inventeur: SOURNAC, Michel, F-31400 Toulouse (FR); BOUGARET, Joël, F-31460 Francarville (FR); RIBEIRO DOS SANTOS, Isabel, F-31600 Eaunes (FR)
(74) Mandataire: Ahner, Francis
(86) Numéro de dépôt international: PCT/EP2008/051972
(87) Numéro de publication internationale: WO 2008/107301

(56) Documents cités:
- EP-A- 0 460 588
- WO-A-01/34121
- WO-A-03/030881
- WO-A-2004/060298
- WO-A-2004/105758
- WO-A-2005/009386
- US-A1- 2003 068 376
- US-A1- 2004 131 661
- US-A1- 2005 186 256
- ANONYMOUS: "Kollicoat IR" TECHNICAL INFORMATION BASF, XX, XX, juillet 2006 (2006-07), pages 1-12, XP002429201

## Description

La présente invention concerne de nouveaux films monocouche à désintégration rapide destinés à l'administration buccale d'une substance pharmaceutique active, aromatique ou cosmétique, leurs procédés de préparation ainsi que leurs utilisations. Lors de l'administration, le film se délite dans la cavité buccale et libère la substance active.

Ainsi, les compositions faisant l'objet de l'invention représentent la solution idéale pour un traitement ambulatoire. Elles permettent l'administration rapide d'une substance active, sans avoir recours à un liquide favorisant la déglutition. Les films tels que décrits dans la présente invention peuvent être placés directement dans la cavité buccale, par exemple sur le palais ou sous la langue, où ils se délitent quasi instantanément.

Une telle propriété est donc avantageuse en particulier pour le traitement de la nausée, souvent éprouvée dans les transports et où la nécessité d'une administration rapide se conjugue souvent à l'impossibilité d'avoir recours à un liquide.

Les compositions de la présente invention apportent également un avantage pour les sujets très jeunes ou âgés, chez qui la déglutition des formes solides pose problème.

Les films à désintégration rapide doivent répondre à des conditions souvent contradictoires : ils doivent d'une part présenter un caractère liant leur permettant d'être mis en forme et, d'autre part, avoir une capacité de délitement quasi instantané. Ils doivent aussi présenter une souplesse et une résistance suffisantes, nécessaires par exemple à leur conditionnement en sachets, en blisters ou en boîtes distributrices.

Les films solubles actuellement utilisés sont principalement constitués de polymères hydrophiles ayant des propriétés filmogènes, et dont l'épaisseur et la surface spécifique permettent d'envisager une désintégration rapide au contact de la salive.

Ces polymères hydrophiles filmogènes sont le plus souvent des glucanes, des gommes naturelles, ou des dérivés de povidone. La combinaison d'au moins deux polymères est généralement souhaitée afin de trouver un compromis entre les principales caractéristiques du film, c'est-à-dire la souplesse, la résistance mécanique et la vitesse de délitement.

Ainsi, dans les films basés sur des polymères cellulosiques, l'association d'au moins deux polymères cellulosiques hydrosolubles est souvent nécessaire. Ceux comprenant l'association de polymères cellulosiques hydrosolubles de masse moléculaire élevée (60000 à 150000 Da) et de polymères cellulosiques hydrosolubles de masse moléculaire faible sont le plus souvent rencontrés. Le premier type de polymère permet de régler les propriétés mécaniques du film, tandis que le second type de polymère permet de faire varier le temps de désintégration.

Par exemple la demande internationale WO 05/039499 décrit une formulation de film pharmaceutique ou cosmétique à désintégration rapide et composée d'une combinaison de type hydroxypropyle cellulose (Klucel JF®) et hydroxypropylméthyle cellulose (Methocel® grades E5, E50, E4M et SGA16M). Toutefois, les films décrits dans cette demande contiennent chacun plusieurs agents plastifiants (polyalcools, ester de sorbitan, esters de l'acide citrique...) qui sont nécessaires à l'obtention de films flexibles et qui rendent la formulation complexe.

Par ailleurs, les polymères de type glucanes comme les pullulanes sont en théorie capables de former des films à désintégration rapide en raison de leur forte solubilité, de leur vitesse de dissolution rapide et de leurs propriétés gustatives. Cependant, leur faible masse moléculaire est un inconvénient qui a pour conséquence de ne pas faciliter la formation de films pour des concentrations inférieures à 20% ; en effet dans ces conditions, la viscosité des mélanges est faible et fait prendre un risque vis-à-vis de l'homogénéité de ces derniers quand, par exemple, une charge insoluble (principe actif en dispersion) est ajoutée au mélange. Il est donc nécessaire d'associer aux pullulanes d'autres polymères naturels comme les carraghénates ou certaines gommes.

Ainsi la demande WO 03/030881 décrit une préparation, sous forme de film comestible comprenant de préférence un glucane et un polymère hydrosoluble, dans un rapport compris entre 40 : 1 et 0,5 : 1. Ce polymère hydrosoluble est caractérisé par une masse moléculaire élevée ce qui permet d'augmenter la viscosité naturellement faible des pullulanes et de stabiliser le mélange lorsqu'il comprend une suspension de principe actif. De préférence ce polymère est un polysaccharide ou une gomme naturelle, faisant partie des alginates, carraghénates, hydroxypropylméthyle cellulose, gomme de caroube, gomme guar, gomme xanthane, dextrans, gomme arabique, gomme gellane, seuls ou en association. Toutefois les compositions décrites dans cette demande contiennent des agents anti-mousse, nécessaires à l'obtention de films dépourvus de bulles d'air. Il est aussi possible d'associer les polymères cellulosiques avec des polymères naturels comme les carraghénates ou certaines gommes. Ainsi, la demande internationale WO 04/105758 décrit une préparation polymérique ternaire, sous forme de film comestible à libération rapide contenant de la triprolidine destinée au traitement des troubles du sommeil, et comprenant de la gomme de xanthane, de l'hydroxypropylméthyle cellulose et du carraghénate.

Les gommes naturelles (gommes guar, gomme de caroube, gomme xanthane, alginates, et carraghénates) ou la polyvinyle pyrrolidone sont souvent considérées comme des polymères stabilisants, qu'il est nécessaire d'ajouter soit aux dérivés cellulosiques, soit aux glucanes pour permettre la réalisation de films. Ces conditions ne sont pas toujours satisfaisantes, ce qui conduit là aussi à recourir à l'ajout d'un composé plastifiant, comme les esters polyoxyéthylénés de sorbitan, les esters d'acides gras et de -glycérol, le glycérol, les esters d'acides gras et de propylène glycol, le propylène glycol, le dibutylsébacate, la triacétine.

Enfin, de nombreuses demandes sont caractérisées par la présence d'un nombre important de constituants. Ainsi la demande internationale WO 03/030883 décrit une préparation sous forme de film comestible basée sur la combinaison complexe d'hydroxypropylméthyle cellulose (Methocel E15®), de polyvinyle pyrrolidone (PVP), d'amidon de maïs (Pure Cote B792®), de gomme xanthane, de surfactant (Cremophor EL®), de plastifiant (propylène glycol) et de préférence un agent masquant le goût.

La demande internationale WO 98/20862 décrit un film à libération immédiate destiné à l'administration orale de principes actifs médicamenteux ou cosmétiques, composé de polymères hydrosolubles, d'un ou plusieurs polyalcools, de plastifiants, de surfactants, d'aromatisants et de colorants. De préférence les polymères sont des dérivés cellulosiques hydrosolubles, comme l'hydroxypropylméthyle cellulose (HPMC), l'hydroxyéthyle cellulose (HEC) et l'hydroxypropyle cellulose (HPC), seuls ou en association. Dans le film sec, la concentration en polymère cellulosique est comprise entre 20 et 75% en masse. D'autres polymères non cellulosiques peuvent également être mis en oeuvre comme la polyvinyle pyrrolidone (PVP), la carboxyméthyle cellulose (CMC), l'alcool Polyvinylique (PVA), l'alginate de sodium, le polyéthylène glycol (PEG), les gommes naturelles comme la gomme xanthane, la gomme adragante, la gomme guar, la gomme d'acacia, la gomme arabique, les polyacrylates hydrodispersibles comme l'acide polyacrylique, le copolymère méthyleméthacrylate, les copolymères carboxyvinyliques.

La demande internationale WO 04/087084 décrit une préparation, sous forme de films comestible à libération rapide composée de polymères filmogènes cellulosiques, plus particulièrement d'un mélange de polymères à forte viscosité et de polymères à faible viscosité présents faible concentration, ce qui présente un avantage économique. Les polymères cellulosiques sont de type hydroxypropylméthyle cellulose (HPMC), de préférence les produits Méthocel®, de la série K ou de la série E, et plus spécifiquement les grades K4M, K100, K3, E50 et E4M. Ici encore, la présence d'agents plastifiants, dans des proportions allant de 0,01% à 30%, est nécessaire à l'obtention de films flexibles et non cassants.

La demande internationale WO 04/045537 décrit une préparation complexe sous forme de film comestible destiné au traitement de la pharyngite, constituée de pullulanes, de gomme guar, de pectines, de gomme xanthane, d'alginates, de gélatine, d'amidons, d'amidons modifiés, de maltodextrines, de gluten, de carboxyméthyle cellulose, de gomme de caroube, de carraghénates.

La demande internationale WO 2004/060298 décrit un film à dissolution rapide destiné à être administré sur la surface d'une muqueuse telle que la cavité buccale et comprenant un copolymère greffé et un ingrédient actif. Le copolymère greffé peut être plus particulièrement le PVA-PEG, tel que le Kollicoat^{®} IR. Les caractéristiques du Kollicoat^{®} IR sont plus particulièrement décrites dans la fiche commerciale de BASF datée de Juillet 2006.

La demande internationale WO 2005/009386 décrit des films oraux à dissolution rapide pour l'administration de substances actives, telles que la nicotine, dans la cavité buccale, Ces films comprennent notamment un polymère entérique ou un polymère filmogène, un dérivé nicotinique et au moins un tampon. Le polymère filmogène peut être un copolymère greffé d'alcool polyvinylique et de polyéthylène glycol, tel que le Kollicoat^{®} IR.

Il était donc nécessaire de concevoir un film comestible à désintégration rapide présentant une formulation simplifiée par rapport aux compositions complexes de l'état de la technique, et qui présente des propriétés rhéologiques (souplesse, résistance mécanique et vitesse de délitement) équivalentes ou améliorées.

L'objet de la présente invention est de proposer de nouveaux films monocouche à désintégration rapide, tels que décrits ci-après et illustrés dans les exemples, dont la matrice est constituée essentiellement de deux polymères hydrosolubles. L'avantage principal des filmes objet de l'invention réside dans le fait qu'ils sont dépourvus de plastifiant, tout en restant suffisamment déformable sans être cassants. Les plastifiants sont en effet souvent hygroscopiques, et rendent délicates la conservation et la manipulation des films obtenus. Leur utilisation n'est donc pas toujours souhaitable. Les films objet de l'invention présentent l'avantage supplémentaire de pouvoir accommoder de façon homogène une substance active insoluble dispersée. Enfin, les films objet de l'invention présentent l'avantage de pouvoir être préférentiellement obtenus grâce à un procédé de mélange conduit sans apport de chaleur extérieur, de façon à préserver au cours de leur procédé de formation la substance active fragile qu'ils contiennent. Cette dernière caractéristique présente également un avantage d'ordre économique.

La présente invention concerne donc un film monocouche à désintégration rapide pour l'administration buccale de substances actives comprenant un support hydrosoluble contenant au moins une substance active, caractérisé en ce qu'il comprend un mélange polymérique d'un agent filmogène hydrophile constitué par un copolymère d'alcool polyvinylique et de polyéthylène glycol (PVA-PEG), d'une substance active et d'un agent hydrophile gélifiant. tel que défini dans la revendication 1.

On entend par film à désintégration rapide, des formes galéniques souples d'une épaisseur inférieure à 100 microns et dont le temps de délitement, dissolution, dispersion ou décomposition dans la cavité buccale reste inférieur à une minute, avantageusement inférieur à-30 secondes, avantageusement de l'ordre-d'une dizaine de secondes. La substance active contenue dans ces films peut ainsi être absorbée par passage de la muqueuse buccale ou sublinguale ou par déglutition.

On entend par température ambiante une température comprise entre 18°C et 25°C, avantageusement comprise entre 20°C et 25°C.

L'agent filmogène hydrophile utilisé dans le cadre de l'invention est un copolymère greffé comprenant un fragment filmogène et un fragment plastifiant, le fragment filmogène étant un alcool polyvinylique (PVA), et le fragment plastifiant étant le polyéthylène glycol (PEG).

Dans une formulation particulièrement avantageuse, l'agent filmogène est le Kollicoat IR® (commercialisé par BASF). Son poids moléculaire est d'environ 45000 Da et il est facilement soluble dans l'eau. Les deux parties du Kollicoat IR® contribuent aux propriétés mécaniques du film obtenu. La partie PVA lui confère des propriétés filmogènes tandis que la partie PEG se comporte comme un plastifiant interne. Ce type d'agent filmogène est déjà connu dans l'art antérieur mais il est habituellement mis en oeuvre pour réaliser des enrobages de comprimés (WO 03/075896), car il permet d'obtenir des préparations de faible viscosité particulièrement appréciées pour leurs propriétés filmogènes.

Toutefois, cette faible viscosité aux concentrations mises en oeuvre (au moins 60% en poids), ne permet pas l'enduction du support afin d'obtenir un film, sans recourir à l'ajout d'un agent gélifiant. De façon surprenante, différentes associations entre des polymères hydrosolubles cellulosiques gélifiants et le Kollicoat IR® ont été effectuées par les inventeurs sans pouvoir obtenir des mélanges homogènes favorisant la stabilité du film lors de l'enduction. Par exemple, afin d'augmenter la viscosité d'une solution de Kollicoat IR®, différents grades d'hydroxypropyle cellulose (HPC) de type Blanose 7HF® ou d'hydroxypropylmethyle cellulose (HPMC) de type Methocel K15M® ont été mis en oeuvre mais les mélanges obtenus sont hétérogènes et conduisent à une floculation impropre à une enduction de qualité.

On entend par enduction, l'étape consistant à étendre le support hydrosoluble sur une surface plane.

Avantageusement, l'agent gélifiant de la formulation n'est pas un dérivé cellulosique. Avantageusement, l'agent gélifiant est un composé de la famille des carraghénates, en particulier le iota-carraghénate.

L'agent gélifiant d'une formulation particulièrement avantageuse est le Gelcarin 379® (commercialisé par FMC Biopolymer), un composé de la famille des iota-carraghénates. La demanderesse a découvert qu'il permettait d'augmenter la viscosité de la solution aqueuse de Kollicoat IR® de manière homogène sans apport de chaleur, en produisant un effet tenseur qui favorise la stabilité du mélange au cours de l'enduction. Le Gelcarin 379® reste dispersé et n'est pas totalement dissous dans le mélange polymérique, car celui-ci n'est pas chauffé jusqu'à l'étape de séchage. Avantageusement, quand il s'agit de carraghénates, la proportion en poids d'agent filmogène à l'agent gélifiant est comprise entre 1 et 10, avantageusement entre 3 et 8.

Dans les formulations basées sur l'association du Kollicoat et du Gelcarin, la quantité en poids d'agent filmogène dans le mélange polymérique représente 10% à 30% du poids total, de préférence de 15% à 20% et la quantité en poids d'agent gélifiant dans le mélange polymérique représente 1% à 8% du poids total, de préférence de 2% à 6%.

Selon une autre variante de l'invention, l'agent gélifiant de la formulation peut être la gomme gellane ou la gomme xanthane.

Dans le cas de la gomme xanthane, la proportion en poids d'agent filmogène à l'agent gélifiant est comprise entre 10 et 200, avantageusement entre 25 et 100.

La quantité en poids de gomme xanthane dans le mélange polymérique est comprise entre 0,1% et 0,5% du poids total et avantageusement comprise entre 0,2% et 0,45% du poids total.

Dans le cas de la gomme gellane, la proportion en poids d'agent filmogène à l'agent gélifiant est comprise entre 100 et 400, avantageusement entre 150 et 300. La quantité en poids de gomme gellane dans le mélange polymérique est comprise entre 0,01% et 0,5% du poids total et avantageusement comprise entre 0,02% et 0,2% du poids total.

Selon une caractéristique avantageuse de l'invention, le mélange polymérique homogène est totalement dépourvu d'agent plastifiant additionnel, c'est-à-dire autre que celui présent dans le polymère filmogène.

Le mélange homogène peut en outre contenir des composants additionnels comme un ou plusieurs tensioactifs. Un tensioactif préféré est le polysorbate 80 (également appelé tween 80).

Toute substance active peut être ajoutée au film, qu'elle soit sous forme dispersée ou dissoute dans le mélange polymérique. Parmi les principes actifs convenant pour réaliser les compositions selon l'invention, on cite à titre indicatif et non limitatif ceux choisis dans le groupe constitué par les médicaments, les arômes ou les additifs alimentaires comme les édulcorants.

Le mélange homogène peut en outre contenir des composants additionnels comme un ou plusieurs tensio actif(s) et/ou un ou plusieurs agent(s) de charge. Un tensio actif préféré est le polysorbate 80 (également appelé "tween 80"). L'agent de charge peut être choisi parmi les charges minérales classiquement utilisées telles que la silice, le talc ou autre silicate, le dioxyde de titane, mais également parmi certains diluants ou lubrifiants pharmaceutiques tels que les stéarates de magnésium, de calcium, ou de zinc.

On cite à titre non limitatif, comme principes actifs médicamenteux, les anti-nauséeux comme par exemple la dompéridone, la méquitazine, la codéine, le chlorhydrate de lopéramide, la combinaison entre le di gluconate de chlorhexidine et la tétracaïne, la nicotine, l'oxybutinine et la cétirizine. Le principe actif est avantageusement la dompéridone.

La présente invention concerne également un procédé pour la fabrication des films à désintégration rapide selon l'invention. Un tel-procédé est caractérisé par la mise en oeuvre des étapes successives suivantes :
a) dispersion dans l'eau des constituants du film ;
b) mélange et homogénéisation de la dispersion ;
c) enduction et séchage du mélange ;
d) découpe des bobines de film et conditionnement.

Avantageusement, la dispersion dans l'eau des constituants du film est réalisée par l'ajout à plus de la moitié de la quantité d'eau et dans l'ordre suivant : de l'agent filmogène, de l'agent tensioactif, du principe actif, du reste de l'eau puis de l'agent gélifiant.

Selon un mode de mise en oeuvre particulier du procédé de fabrication selon l'invention, le film est obtenu sans apport de chaleur extérieur par mélange d'un agent filmogène hydrophile constitué par un copolymère d'alcool polyvinylique et de polyéthylène glycol (PVA-PEG) de viscosité comprise entre 1 et 250 mPa.s à température ambiante et d'une substance active avec un agent hydrophile gélifiant en présence d'eau pour obtenir un mélange polymérique aqueux homogène, la proportion d'agent filmogène à l'agent gélifiant étant déterminée pour conférer audit mélange polymérique aqueux homogène une viscosité à température ambiante comprise entre 250 mPa.s et 15000 mPa.s, de préférence comprise entre 1000 mPa.s et 10000 mPa.s, plus préférentiellement encore comprise entre 1500 mPa.s et 9000 mPa.s, avant la formation du film par une technique classique d'enduction et séchage.

Les films obtenus après l'étape de séchage peuvent être découpés, soit en unités individuelles de taille comprise entre 4 cm² et 10 cm², soit être enroulés sous forme de rouleaux, soit pliés. Dans chacune de ces situations, les films après enduction sont accompagnés ou non d'un support détachable.

Ces films peuvent être conditionnés de différentes manières, en sachets individuels, en plaques prédécoupées, en blisters individuels ou en boîtes distributrices.

L'invention va maintenant être illustrée à titre non limitatif par les exemples suivants. Plusieurs films à désintégration rapide, contenant de la dompéridone ou un autre principe actif, ont été préparés à partir d'un mélange polymérique aqueux homogène de Kollicoat IR® et de Gelcarin 379®. Les proportions du mélange ainsi que les propriétés physiques des films obtenus après enduction et séchage sont réunies dans les Tableaux 1 (essais laboratoire) et 2 (essais pilote).

### Exemple 1 :

Le Kollicoat IR® a été introduit dans 70% de la quantité d'eau purifiée sous agitation. L'agitation a été maintenue jusqu'à dissolution du Kollicoat IR®. Celle-ci générant beaucoup de bulles, il est possible soit de le dissoudre sous vide, soit de laisser reposer la solution (sa viscosité étant est très faible celle-ci débulle seule). Le tween 80 a été incorporé à la solution sous agitation, puis les arômes (extrait condensé de réglisse et huile essentielle de menthe poivrée) et l'édulcorant (acésulfame potassique) ont été ajoutés. L'agitation a été poursuivie jusqu'à solubilisation complète des poudres. La dompéridone a été introduite sous agitation jusqu'à sa dispersion dans le mélange, puis le reste d'eau (30%) a été ajouté. Le Gelcarin 379® a été incorporé à la suspension de dompéridone sous agitation, afin d'éviter la formation d'agrégats. Des aliquots de mélange ont ensuite été enduits sur un support polyester et séchés dans un équipement de type Lab Dryer Coater Mathis. Les surfaces enduites ont été découpées à l'aide d'une presse manuelle en unités de 6 cm², puis conditionnées manuellement dans des sachets étanches.

**Tableau 1. Films à désintégration rapide à base d'un mélange Kollicoat IR/Gelcarin préparés à la paillasse.**

| **Film** | **1** | **2** | **3** |
|---|---|---|---|
| **% m/m mélange** | | | |
| | | | |
| Dompéridone | 6 | 9,7 | 8,6 |
| Kollicoat IR | 15 | 17 | 15,8 |
| Gelcarin 379 | 5 | 3 | 2,7 |
| Tween 80 | 0,2 | 0,2 | 0,19 |
| Acesulfame potassique | 0,5 | 0,5 | 0,43 |
| Arômes | 1,5 | 1,65 | 0,8 |
| Eau purifiée | qsp | qsp | qsp |
| **Viscosité (mPa.s)¹** | 1540 | 1660 | 1980 |
| | | | |

| **Propriétés du film** | | | |
|---|---|---|---|
| | | | |
| Surface (cm²) | 6 | 6 | 6 |
| Epaisseur (µm) | 65 | 40 | 40 |
| Grammage (g/m²) | 87,3 | 60 | 63 |
| Teneur en eau (%) | 8,6 | 8,8 | 9 |
| Surface spécifique (cm²/g) | 220 | 334 | 334 |
| Force de rupture (N) | 6,5 | 12,9 | 16,50 |
| Elongation (%) | 3,2 | 3,1 | 4,3 |
| Temps de désintégration²(sec) | 12 | 7 | 15 |

| | | | |
|---|---|---|---|
| ¹Brookfield LVT3, 22°C, 20 trs/min, 50 ml d'eau à 37°C. | | | |

### Exemple 2 :

Pour la réalisation de lots de taille pilote, les composants ont été introduits dans une cuve dans l'ordre suivant : plus de la moitié de la quantité d'eau, Kollicoat IR®, édulcorants et arômes (extrait condensé de réglisse et huile essentielle de menthe poivrée), tween 80 et dompéridone. Le prémélange ainsi obtenu a été homogénéisé par passages successifs dans une filière puis réintroduit dans la cuve principale. Les composants restants (Gelcarin 379® et la quantité résiduelle d'eau) ont été rajoutés au prémélange, puis le tout a été agité. Le mélange final a été homogénéisé au cours de trois passages successifs en filière, puis laissé au repos sous une pression réduite d'azote, afin de permettre un débullage de bonne qualité avant d'envisager l'étape d'enduction. Le mélange a été transféré à l'aide d'une pompe vers une ligne d'enduction pilote, constituée d'un poste d'enduction, de trois zones successives de séchage et d'un poste de contrecollage. La laize et la vitesse d'enduction sont respectivement de 0,85 m/min et 17 cm. Un gradient de température a été mis en place, d'une part pour éviter une dégradation du principe actif sous l'effet de la chaleur, et d'autre part pour cibler une quantité résiduelle en eau dans le film, compatible avec les caractéristiques mécaniques de ce dernier (un film trop sec sera trop cassant et impropre à toute manipulation). Après une période de maturation, la bobine de film a été découpée et conditionnée à l'aide d'équipements dédiés.

**Tableau 2. Films à désintégration rapide à base d'un mélange Kollicoat IR/Gelcarin préparés en ligne_pilote.**

| **Film** | **4** | **5** | **6** |
|---|---|---|---|
| **% m/m mélange** | | | |
| | | | |
| Dompéridone | 5,75 | 9,8 | 9,9 |
| Kollicoat IR | 15,6 | 18,2 | 18,2 |
| Gelcarin 379 | 5,2 | 3,1 | 3,1 |
| Tween 80 | 0,16 | 0,21 | 0,21 |
| Acesulfame potassique | 0,16 | 0,5 | 0,5 |
| Aromes | 1,37 | 0,41 | 0,4 |
| Eau purifiée | qsp | qsp | qsp |
| **Viscosité (mPa.s)¹** | **4400** | **7964** | **8496** |
| | | | |

| **Propriétés du film** | | | |
|---|---|---|---|
| | | | |
| Surface (cm²) | 6 | 6 | 6 |
| Epaisseur (µm) | 65 | 46 | 45 |
| Grammage (g/m²) | 88,4 | 59 | 58 |
| Teneur en eau (%) | 9,6 | 7,3 | 7,8 |
| Force de rupture (N) | 7,5 | 11,1 | 9,4 |
| Elongation (%) | 5,8 | 3,85 | 4 |
| Temps de désintégration²(sec) | 21,5 | 7 | 7 |
| Temps de désintégration³(sec) | 21,6 | 10 | 10 |

| | | | |
|---|---|---|---|
| ¹ Brookfield, LV4, 25°C, 50 trs/min, ² 50 ml d'eau à 37°C, Sotax, 800 ml d'eau à 37°C | | | |

### Exemple 3 :

D'autres films à désintégration rapide ont été préparés à partir d'un mélange polymérique aqueux homogène de Kollicoat IR® et de gomme de gellane ou de gomme de xanthane, en suivant le procédé de l'Exemple 1. Les résultats sont rassemblés dans le Tableau 3.

**Tableau 3. Films à désintégration rapide à base d'un mélange Kollicoat IR/gomme gellane (Kelcogel) ou gomme xanthane (Kelgum), et contenant de la dompéridone.**

| **Film** | **7** | **8** |
|---|---|---|
| % m/m mélange | | |
| Dompéridone | 8,2 | 8,2 |
| Kollicoat IR | 18,2 | 18,2 |
| Kelgum | 0,4 | |
| Kelcogel LT100 | | 0,075 |
| Tween 80 | 0,18 | 0,17 |
| Eau purifiée | qsp | qsp |
| **Viscosité (mPa.s)¹** | **1900** | **2400** |
| | | |

| **Propriétés du film** | | |
|---|---|---|
| | | |
| Surface (cm²) | 6 | 6 |
| Grammage (g/m²) | 57,09 | 60,88 |
| Teneur en eau (%) | 10 | 10,24 |

| | | |
|---|---|---|
| ¹ Brookfield, LV4, spindle 2, 22,5°C, 10trs/min | | |

### Exemple 4 :

Une variété de principes actifs a pu être intégrée dans les films selon l'invention. Les compositions des films obtenus selon le mode opératoire de l'Exemple 1 décrit précédemment, sont rassemblées dans le Tableau 4.

**Tableau 4. Films à désintégration rapide comprenant différents principes actifs.**

| **Film** | **9** | **10** | **11** | **12** | **13** |
|---|---|---|---|---|---|
| **Composition (mg)** | | | | | |
| | | | | | |
| Digluconate de chlorhexidine | 2,53 | | | | |
| Tetracaïne | 0,19 | | | | |
| Méquitazine | | 10 | | | |
| Caféine | | | 10 | | |
| Lopéramide HCl | | | | 2 | |
| Codéine Phophate hémihydrate | | | | | 18,68 |
| Kollicoat IR | 21,88 | 18,49 | 18,49 | -25,32 | 17,73 |
| lota-carraghénate | 1,67 | 3,12 | 3,14 | 4,30 | 2,99 |
| Polysorbate 80 | 0,25 | 0,21 | 0,21 | 0,22 | 0,33 |
| Acesulfame potassique | 0,46 | 0,51 | 0,51 | 0,51 | 0,63 |
| Glycyrrhizinate d'ammonium | 0,19 | 0,22 | 0,21 | 0,21 | 0,27 |
| Arômes | - | 0,21 | 0,20 | 0,20 | 0,33 |
| Eau purifiée | q.s.p | q.s.p | q.s.p | q.s.p | q.s.p |
| Total | 30,0 | 36,0 | 36,0 | 36,0 | 45,0 |

### Exemple 5

Un autre film à désintégration rapide qui a donné des résultats particulièrement satisfaisants a été préparé à partir d'un mélange polymérique aqueux homogène d'un copolymère PVA-PEG (Kollicoat IR®) et d'un iota-carraghénate (Gelcarine 379®) en suivant le procédé décrit précédemment.

Les résultats ont été rassemblés dans le Tableau 5.

**Tableau 5. Composition unitaire d'un film à désintégration rapide à base de dompéridone.**

| **Film** | **14** | |
|---|---|---|
| **Produit fini** | mg | % m/m |
| | | |
| Dompéridone | 10,0 | 30,3 |
| Kollicoat IR | 16,85 | 51,0 |
| Gelcarine 379 | 2,73 | 8,3 |
| Tween 80 | 0,20 | 0,6 |
| Acesulfame potassique | 0,50 | 1,51 |
| Ammonium glycyrrhizate | 0,20 | 0,6 |
| Extrait de réglisse | 0,04 | 0,12 |
| Menthe poivrée | 1,0 | 3,03 |
| Eau | qsp | |
| | | |
| Total | 33,0 | 100,0 |

## Revendications

1. Film monocouche à désintégration rapide pour l'administration buccale de substances actives, comprenant un support hydrosoluble contenant au moins une substance active, **caractérisé en ce qu'**il comprend un mélange polymérique d'un agent filmogène 1 constitué par une copolymère d'alcool polyvinylique et de polyéthylène glycol (PVA-PEG), d'une substance active et d'un agent hydrophile gélifiant, l'agent hydrophile gélifiant est choisi dans la liste constituée par les carraghénates de préférence le iota-carraghénate, la gomme gellane et la gomme de xanthane.

2. Film selon la revendication 1, **caractérisé en ce que** le mélange polymérique est dépourvu d'agent plastifiant additionnel.

3. Film selon la revendication 1, **caractérisé en ce que** la proportion en poids. d'agent filmogène à l'agent hydrophile gélifiant est comprise entre 1 et 10 quand ce dernier appartient à la famille des carraghénates, de préférence comprise entre 3 et 8.

4. Film selon la revendication 3, **caractérisé en ce que** la quantité en poids d'argent filmogène dans le mélange polymérique représente 10% à 30% du poids total, de préférence de 13% à 20%, et la quantité en poids d'agent hydrophile gélifiant dans le mélange polymérique représente 1% à 8% du poids total, de préférence de 3% à 6%.

5. Film selon la revendication 1, **caractérisé en ce que** lorsque l'agent gélifiant est une gomme de xanthane, la proportion en poids d'agent filmogène à l'agent hydrophile gélifiant est comprise entre 10 et 200, de préférence comprise entre 25 et 100.

6. Film selon la revendication 5, **caractérisé en ce que** la quantité en poids de gomme xanthane dans le mélange polymérique est comprise entre 0,1% et 0,5% du poids total, de préférence comprise entre 0,2% et 0,45% du poids total.

7. Film selon la revendication 1, **caractérisé en ce que** lorsque l'agent gélifiant est une gomme gellane, la proportion en poids d'agent filmogène à l'agent hydrophile gélifiant est comprise entre 100 et 400, de préférence entre 150 et 300.

8. Film selon la revendication 7, **caractérisé en ce que** et la quantité en poids d'agent hydrophile gélifiant dans le mélange polymérique est comprise entre 0,01% est 0,5% du poids total, de référence comprise entre 0,02% et 0,2% du poids total.

9. Film selon l'une quelconque des revendication précédentes, **caractérisé en ce que** la substance active est choisie dans le groupe comprenant la dompéridone, la méquitazine, la codéine, le chlorhydrate de lopéramide, la combinaison entre le di gluconate de chlorhexidine et la tétracaïne, la nicotine, l'oxybutinine et la cétirizine, et est de préférence la dompéridone.

10. Film selon l'une des revendications précédentes, **caractérisé en ce qu'**il comprend en outre un ou plusieurs agent(s) de charge et/ou un ou plusieurs agent(s) tensio actifs.

11. Film selon l'une quelconque des revendications précédentes, **caractérisé en ce qu'**il comprend le polysorbate 80 à titre d'agent tensio actif.

12. Procédé pour la fabrication d'un film selon l'une quelconque des revendications précédentes, **caractérisé par** la mise en oeuvre des étapes successives suivantes :
a) dispersion dans l'eau des constituants du film ;
b) mélange et homogénéisation de la dispersion ;
c) enduction et séchage du mélange.

13. Procédé selon la revendication 12, **caractérisé en ce que** la dispersion dans l'eau des constituants du film est réalisée par l'ajout à plus de la moitié de la quantité d'eau et dans l'ordre suivant : de l'agent filmogène, de l'agent tensioactif, du principe actif, du reste de l'eau puis de l'agent hydrophile gélifiant.

14. Procédé selon l'une des revendications 12 et 13, **caractérisé en ce que** le film est obtenu sans apport de chaleur extérieur par mélange d'un agent filmogène hydrophile constitué par un coplymère d'alcool polyvinylique et de polyéthylène glycol (PVA-PEG) de viscosité comprise entre 1 et 250 mPa.s à température ambiante et d'une substance active avec un agent hydrophile gélifiant en présence d'eau pour obtenir un mélange polymérique aqueux homogène, la proportion d'agent filmogène à l'agent gélifiant étant déterminée pour conférer audit mélange polymérique aqueux homogène une viscosité à température ambiante comprise entre 250 mPa.s et 15000 mPa.s, de préférence comprise entre 1000 mPa.s et 10000 mPa.s, plus préférentiellement encore comprise entre 1500 mPa.s et 9000 mPa.s, avant la formation du film par une technique classique d'enduction et séchage.

15. Film selon l'une quelconque des revendications 1 à 11 en tant que médicament.

## Claims

1. Rapid disintegration monolayer film for the oral administering of active substance, comprising a water-soluble support containing at least one active substance, **characterized in that** it comprises a polymeric mixture of a hydrophilic film-forming agent consisting of a copolymer of polyvinyl alcohol and polyethylene glycol (PVA-PEG), an active substance and a hydrophilic gelling agent, the hydrophilic gelling agent is chosen from the list comprising carrageenans preferably iota-carrageenan, gellan gum and xanthan gum.

2. The film according to claim 1, **characterized in that** the polymeric mixture is devoid of an additional plasticizer.

3. The film according to claim 1, **characterized in that** the proportion by weight of film-forming agent to hydrophilic gelling agent lies between 1 and 10 when the latter belongs to the carrageenan family, preferably between 3 and 8.

4. The film according to claim 3, **characterized in that** the quantity by weight of film-forming agent in the polymeric mixture represents 10% to 30% of the total weight, preferably 13% to 20%, and the quantity by weight of hydrophilic gelling agent in the polymeric mixture represents 1% to 8% of the total weight, preferably 2% to 6%.

5. The film according to claim 1, **characterized in that** when the gelling agent is a xanthan gum, the proportion by weight of film-forming agent to hydrophilic gelling agent lies between 10 and 200, preferably between 25 and 100.

6. The film according to claim 5, **characterized in that** the quantity by weight of xanthan gum in the polymeric mixture lies between 0.1% and 0.5% of the total weight, preferably between 0.2% and 0.45% of the total weight.

7. The film according to claim 1, **characterized in that** when the gelling agent is a gellan gum, the proportion by weight of film-forming agent to hydrophilic gelling agent lies between 100 and 400, preferably between 150 and 300.

8. The film according to claim 7, **characterized in that** quantity by weight of hydrophilic gelling agent in the polymeric mixture lies between 0.01% and 0.5% of the total weight, preferably between 0.02% and 0.2% of the total weight.

9. The film according to any of the preceding claims, **characterized in that** the active substance is chosen from the group comprising domperidone, mequitazine, codein, loperamide hydrochloride, the combination of chlorhexidine digluconate and tetracaine, nicotine, oxybutinine and cetirizine, and is preferably domperidone.

10. The film according to any of the preceding claims, **characterized in that** it further contains one or more fillers and/or one or more surfactants.

11. The film according to any of the preceding claims, **characterized in that** it contains polysorbate 80 as surfactant.

12. Method to produce a film according to any of the preceding claims, **characterized by** conducting the following successive steps:
a) dispersing the film constituents in water,
b) mixing and homogenizing the dispersion,
c) coating and drying the mixture.

13. The method according to claim 12, **characterized in that** the dispersion in water of the film constituents is obtained by adding to more than one half of the quantity of water and in the following order: film-forming agent, surfactant, active ingredient, remainder of the water, then the hydrophilic gelling agent.

14. The method according to either of claims 12 and 13, **characterized in that** the film is obtained without applying any external heat by mixing a hydrophilic film-forming agent, consisting of a copolymer of polyvinyl alcohol and polyethylene glycol (PVA-PEG) of viscosity between 1 and 250 mPa.s at ambient temperature, with an active substance and a hydrophilic gelling agent in the presence of water to obtain a homogeneous aqueous polymeric mixture, the proportion of film-forming agent to gelling agent being determined so as to impart to said homogeneous aqueous polymeric mixture a viscosity at ambient temperature of between 250 mPa.s and 15,000 mPa.s, preferably between 1,000 mPa.s and 10,000 mPa.s, further preferably between 1,500 mPa.s and 9,000 mPa.s before forming of the film using a conventional coating and drying technique.

15. The film according to any of claims 1 to 11 as medicinal product.

## Patentansprüche

1. Schnell zerfallende einschichtige Folie für die bukkale Verabreichung von Wirkstoffen, umfassend einen wasserlöslichen Träger, der mindestens einen Wirkstoff enthält, **dadurch gekennzeichnet, dass** sie eine polymere Mischung eines hydrophilen filmbildenden Mittels, das aus einem Copolymer von Polyvinylalkohol und Polyethylenglycol (PVA-PEG) besteht, eines Wirkstoffes und eines hydrophilen Gelierungsmittels umfasst, wobei das hydrophile Gelierungsmittel aus der Liste ausgewählt ist, die aus Carragheenaten, vorzugsweise lota-Carragheenat, Gellangummi und Xanthangummi besteht.

2. Folie nach Anspruch 1, **dadurch gekennzeichnet, dass** die polymere Mischung keinen zusätzlichen Weichmacher enthält.

3. Folie nach Anspruch 1, **dadurch gekennzeichnet, dass** das Gewichtsverhältnis von filmbildendem Mittel zu hydrophilem Gelierungsmittel zwischen 1 und 10 einschließlich liegt, wenn das letztgenannte der Familie der Carragheenate angehört, vorzugsweise zwischen 3 und 8 einschließlich.

4. Folie nach Anspruch 3, **dadurch gekennzeichnet, dass** die Menge bezüglich Gewicht des filmbildenden Mittels in der polymeren Mischung 10 % bis 30 % des Gesamtgewichts, vorzugsweise 13 % bis 20 % ausmacht, und die Menge bezüglich Gewicht an hydrophilem Gelierungsmittel in der polymeren Mischung 1 % bis 8 % des Gesamtgewichts, vorzugsweise 2 % bis 6 % ausmacht.

5. Folie nach Anspruch 1, **dadurch gekennzeichnet, dass**, wenn das Gelierungsmittel ein Xanthangummi ist, das Gewichtsverhältnis von
filmbildendem Mittel zu hydrophilem Gelierungsmittel zwischen 10 und 200 einschließlich, vorzugsweise zwischen 25 und 100 einschließlich liegt.

6. Folie nach Anspruch 5, **dadurch gekennzeichnet, dass** die Menge bezüglich Gewicht an Xanthangummi in der polymeren Mischung zwischen 0,1 % und 0,5 % einschließlich des Gesamtgewichts, vorzugsweise zwischen 0,2 % und 0,45 % einschließlich des Gesamtgewichts liegt.

7. Folie nach Anspruch 1, **dadurch gekennzeichnet, dass**, wenn das Gelierungsmittel ein Gellangummi ist, das Gewichtsverhältnis von filmbildendem Mittel zu hydrophilem Gelierungsmittel zwischen 100 und 400 einschließlich, vorzugsweise zwischen 150 und 300 einschließlich liegt.

8. Folie nach Anspruch 7, **dadurch gekennzeichnet, dass** die Menge bezüglich Gewicht an hydrophilem Gelierungsmittel in der polymeren Mischung zwischen 0,01 % und 0,5 % einschließlich des Gesamtgewichts, vorzugsweise zwischen 0,02 % und 0,2 % einschließlich des Gesamtgewichts liegt.

9. Folie nach irgendeinem der vorangehenden Ansprüche, **dadurch gekennzeichnet, dass** der Wirkstoff ausgewählt ist aus der Gruppe, die Domperidon, Mequitazin, Codein, Loperamid-Hydrochlorid, die Kombination zwischen dem Digluconat von Chlorhexidin und Tetracain, Nikotin, Oxybutinin und Cetirizin umfasst, und vorzugsweise Domperidon ist.

10. Folie nach einem der vorangehenden Ansprüche, **dadurch gekennzeichnet, dass** sie darüber hinaus einen oder mehrere Füllstoffe und/oder ein oder mehrere Tenside umfasst.

11. Folie nach irgendeinem der vorangehenden Ansprüche, **dadurch gekennzeichnet, dass** sie Polysorbat 80 als Tensid umfasst.

12. Verfahren zur Herstellung einer Folie nach irgendeinem der vorangehenden Ansprüche, **gekennzeichnet durch** die Durchführung der folgenden aufeinander folgenden Schritte:
a) Dispergieren der Bestandteile des Films in Wasser;
b) Mischen und Homogenisieren der Dispersion;
c) Auftragen und Trocknen der Mischung.

13. Verfahren nach Anspruch 12, **dadurch gekennzeichnet, dass** die Dispergierung der Bestandteile der Folie im Wasser durch Zugabe zu mehr als der Hälfte der Wassermenge und in der folgenden Reihenfolge: filmbildendes Mittel, Tensid, Wirkstoff, Rest des Wassers, dann hydrophiles Gelierungsmittel, durchgeführt wird.

14. Verfahren nach einem der Ansprüche 12 und 13, **dadurch gekennzeichnet, dass** die Folie ohne Zufuhr äußerer Wärme durch Mischen eines hydrophilen filmbildenden Mittels, das aus einem Copolymer von Polyvinylalkohol und Polyethylenglycol (PVA-PEG) mit einer Viskosität zwischen 1 und 250 mPa.s einschließlich bei Umgebungstemperatur besteht, und eines Wirkstoffs mit einem hydrophilen Gelierungsmittel in Anwesenheit von Wasser zum Erhalt einer wässrigen homogenen polymeren Mischung erhalten wird, wobei das Verhältnis von filmbildendem Mittel zu Gelierungsmittel so bestimmt wird, dass der wässrigen homogenen polymeren Mischung vor der Bildung der Folie durch eine klassische Technik des Auftragens und Trocknens eine Viskosität bei Umgebungstemperatur zwischen 250 mPa.s und 15000 mPa.s einschließlich, vorzugsweise zwischen 1000 mPa.s und 10000 mPa.s einschließlich, noch bevorzugter zwischen 1500 mPa.s und 9.000 mPa.s einschließlich verliehen wird.

15. Folie nach irgendeinem der Ansprüche 1 bis 11 als Medikament.
